# EUROPEAN PATENT APPLICATION

(11) **EP 4 454 573 A1**
(43) Date of publication of application: **30.10.2024**
(21) Application number: 24172876.5
(22) Date of filing: 26.04.2024
(51) Int. Cl.: A61B 17/22, A61M 25/00

(54) **DOUBLE FLOW CATHETER**

(30) Priority: 28.04.2023 WO PCT/EP2023/061391
(71) Applicant: Balt Innovation SAS, 95160 Montmorency (FR)
(72) Inventor: BABEAU, Olivier, 68420 Voegtlinshoffen (FR)
(74) Representative: Marks & Clerk LLP

(57) **Abstract**

Systems, catheters, and methods are generally directed to facilitating rapid access to thrombi formed in tortuous vasculature and use of dual flows (e.g., aspiration and fluid injection) in coordination with one another and/or one or more devices (e.g., stent retrievers) to remove the thrombi efficiently, as may be useful for achieving successful patient outcomes in the treatment of a variety of disease conditions, such as acute ischemic stroke.

## Description

### BACKGROUND

Thrombus formation occurs when the flow of blood slows down or is impeded or there is damage inside a blood vessel. Blood cells pool together to form a clot, narrowing or blocking the passage of blood and depriving cells of oxygen-rich blood necessary for survival. Acute ischemic stroke is a sudden blockage of blood flow to the brain cells and is typically caused by thrombus or other emboli lodging in one of the blood vessels supplying the brain. Without quick resolution of the blockage, permanent neurological deficit or death may result.

Known treatment methods for treating occlusions include treatment with a thrombectomy device and an aspiration catheter in combination with one another to increase success in thrombus removal. For use in cerebral arteries, however, aspiration catheters need to be rapidly navigable through tortuous neurovasculature and, ultimately, to the thrombus while also having a large enough lumen to extract the thrombus from the treatment site to treat the acute ischemic stroke.

Given the critical importance of time in treating certain types of blockages, particularly in cerebral vasculature, there is a need for aspiration catheters that facilitate faster and more effective thrombus removal.

### SUMMARY

Systems, catheters, and methods are generally directed to facilitating rapid access to thrombi formed in tortuous vasculature and use of dual flows (e.g., aspiration and fluid injection) in coordination with one another and/or in coordination with other devices (e.g., stent retrievers) to remove the thrombi efficiently, as may be useful for achieving successful patient outcomes in the treatment of a variety of disease conditions, such as acute ischemic stroke.

According to one aspect, a double flow catheter for removal of an occlusion within a vessel may include a first tube having a proximal portion and a distal portion and defining first lumen therebetween, the distal portion defining a first orifice in fluid communication with the first lumen, the first lumen having a first radial cross-sectional area, and a second tube defining a second lumen parallel to the first lumen, the second tube including a nose defining a portion of the second lumen distal to the first lumen, the nose defining a second orifice in fluid communication with the second lumen, the second tube and the first tube coupled to one another and collectively defining a body proximal to the nose, and the second lumen having a second radial cross-sectional area less than the first radial cross-sectional area.

In certain implementations, the body may have an overall bend radius that decreases from the proximal portion to the distal portion of the first tube. In some instances, the overall bend radius may decrease according to a gradient along at least a portion of a longitudinal dimension of the body.

In some implementations, the first lumen may define a first longitudinal axis, the second lumen defines a second longitudinal axis, and the first longitudinal axis and the second longitudinal axis are parallel to one another and radially spaced from one another at least from the proximal portion of the first tube to the distal portion of the first tube.

In certain implementations, the second radial cross-sectional area of the second lumen may be proximal to the nose of the second tube. The second radial cross-sectional area of the second lumen may be along the portion of the second lumen defined by the nose of the second tube. In some instances, the second radial cross-sectional area may be substantially constant along a longitudinal dimension of the second lumen.

In some implementations, the first radial cross-sectional area of the first lumen may be substantially constant along a longitudinal dimension of the first lumen.

In certain implementations, at least from the proximal portion to the distal portion of the first tube, a ratio of the first radial cross-sectional area to the second radial cross-sectional area is less than about 11:1.

In some implementations, the second radial cross-sectional area may be greater than or equal to 0.1 mm² and less than or equal to 0.25 mm².

In certain implementations, the shape of the first radial cross-sectional area of the first lumen and the shape of the second radial cross-sectional area of the second lumen may each include a respective arcuate segment. In some instances, the shape of the first radial cross-sectional area of the first lumen may be elliptic from the proximal portion to the distal portion of the first tube. Further, or instead, the shape of the second radial cross-sectional area of the second lumen may be elliptic

In some implementations, the second lumen maty be fluidically isolated from the first lumen.

In certain implementations, the body may have an outer surface continuous from the proximal portion to the distal portion of the first tube. In some instances, the outer surface of the body may have a maximum radial dimension greater than about 1 mm and less than about 6 mm. In certain instances, from the proximal portion to the distal portion of the first tube, a circumference of the outer surface of the body has an oblong shape. In some instances, from the proximal portion to the distal portion of the first tube, the body may be more flexible in a first plane defined by a minor dimension of the oblong shape and a longitudinal axis defined by the first lumen than in a second plane defined by a major dimension of the oblong shape and the longitudinal axis defined by the first lumen. Additionally, or alternatively, from the proximal portion to the distal portion of the first tube, the circumference of the outer surface may be stadium-shaped. Further, or instead, from the proximal portion to the distal portion of the first tube, a circumference of the outer surface may be oval. Still further, or in the alternative, the outer surface of the body may include a hydrophilic coating.

In some implementations, the double flow catheter may further include a first lining in the first lumen and a second lining in the second lumen, wherein the first lining and the second lining may each hydrophobic. For example, at least one of the first lining or the second lining may include polytetrafluoroethylene (PTFE), a polymer having a lower surface energy than PTFE, or a combination thereof.

In certain implementations, the first tube may have a first radial compressive strength of at least about 1050 mmHg along the first lumen, and the second tube has a burst pressure of at least about 1050 mmHg along the second lumen at least proximal to the nose of the second tube.

In some implementations, at least one of the first tube or the second tube may have a burst pressure of greater than 37500 mmHg.

In certain implementations, the first tube and the second tube may be fused to one another from the proximal portion to the distal portion of the first tube.

In some implementations, the first tube may have a first polymeric composition profile varying in polymeric composition in an axial direction along the first tube, and the second tube has a second polymeric composition profile varying in polymeric composition in an axial direction along the second tube. In certain instances, the first polymeric composition profile and the second polymeric composition profile may have the same composition in at least one axial position along a longitudinal dimension of the body from the proximal portion to the distal portion of the first tube. Additionally, or alternatively, the nose of the second tube may include a proximal section and a distal section, the proximal section of the nose is adjacent to the distal portion of the first tube, and the distal section of the nose is distally away from the distal portion of the first tube and defines the second orifice, and the second polymeric composition profile is softest along at least a portion of the nose between the proximal section and the distal section of the nose. In certain instances, composition of the second polymeric composition profile may be constant in an axial direction from the proximal section to the distal section of the nose. Further, or instead, the nose may have a longitudinal dimension of greater than about 15 mm and less than about 75 mm from the proximal section to the distal section. Additionally, or alternatively, from the proximal section to the distal section, a maximum outer dimension of the nose is greater than about 0.4 mm and less than about 1.0 mm. Still further, or in the alternative, from the proximal section to the distal section, an outer circumference of the nose is circular. In some instances, the portion of the second lumen defined by the nose may accommodate passage of a micro guidewire from the proximal section of the nose through the second orifice defined by the distal section of the nose. For example, the portion of the second lumen defined by the nose may have a maximum radial inner dimension of greater than about 0.2 mm and less than about 0.6 mm. Further, or instead, the nose may define a plurality of apertures from the proximal section to the distal section.

In certain implementations, the body may include one or more reinforcement materials from the proximal portion to the distal portion of the first tube. In some instances, density of the one or more reinforcement materials per unit of axial length may decrease in an axial direction from the proximal portion to the distal portion of the first tube. In certain instances, the one or more reinforcement materials may include one or more wires. For example, at least one of the one or more wires may be wrapped in a pattern with decreasing pitch, have decreasing cross-sectional area, or a combination thereof along the body in the axial direction from the proximal portion to the distal portion of the first tube. Further, or instead, the one or more wires may form a coil, an overlapping pattern, an interwoven pattern, or a combination thereof about at least one of the first tube or the second tube. Still further, or in the alternative, one or more reinforcement materials may include stainless steel, nitinol, tungsten, polymeric fibers, or a combination thereof.

In some implementations, the double flow catheter may further include a plurality of radiopaque markers, wherein at least one of the plurality of radiopaque markers is disposed along the distal portion of the first tube, and at least one of the plurality of radiopaque markers is disposed along the nose of the second tube. For example, the plurality of radiopaque markers may include rings, dots, or a combination thereof of radiopaque material. Additionally, or alternatively, the radiopaque material may include one or more radiopaque polymers.

Other aspects, features, and advantages will be apparent from the description and drawings, and from the claims.

### BRIEF DESCRIPTION OF THE DRAWINGS

FIG. 1A is a schematic representation of a system for clot removal, with the system including a double flow catheter, a hub, a vacuum source, and a liquid source.
FIG. 1B is an end view of a cross-section of the double flow catheter of FIG. 1A, with the cross-section taken through a first tube and a second tube along 1B-1B in FIG. 1A.
FIG. 1C is an end view of a cross-section of the double flow catheter of FIG. 1A, with the cross-section taken through a nose of the second tube of FIG. 1B along 1C-1C in FIG. 1A.
FIG. 1D is a side, cross-sectional view of the double flow catheter of FIG. 1A, shown close-up along area of detail 1D in FIG. 1A.
FIG. 2A is a schematic representation of brain vascularization, with the brain vascularization represented in the coronal plane and shown with a thrombus, in one of the segments of a middle cerebral artery, causing an ischemic stroke in the patient.
FIG. 2B is schematic representation of a portion of the double flow catheter of the system of FIG. 1A, shown with injection and aspiration applied at the treatment site of the clot in the middle cerebral artery of FIG. 2A for removal of the clot to treat the ischemic stroke in the patient.
FIG. 3 is an end view of a cross-section of a double flow catheter including a first tube and a second tube, the first tube defining a first lumen a matching a shape of the second tube.
FIG. 4 is an end view of a cross-section of a double flow catheter including a first tube and a second tube, the first tube defining a first lumen, and the second tube disposed in the first lumen of the first tube.
FIG. 5 is a side cross-sectional view of a nose of a double flow catheter, with the nose defining a plurality of apertures.

Like reference symbols in the various drawings indicate like elements.

### DESCRIPTION

Embodiments will now be described with reference to the accompanying figures. The foregoing may, however, be embodied in many different forms and should not be construed as limited to the embodiments set forth herein.

All documents mentioned herein are hereby incorporated by reference in their entirety. References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and, similarly, the term "and" should generally be understood to mean "and/or."

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately," or the like, when accompanying a numerical value, are to be construed as indicating a deviation as would be appreciated by one of ordinary skill in the art to operate satisfactorily for an intended purpose. Ranges of values and/or numeric values are provided herein as examples only, and do not constitute a limitation on the scope of the described embodiments. The use of any and all examples, or exemplary language ("e.g.," "such as," or the like) provided herein, is intended merely to describe the embodiments better and does not pose a limitation on the scope of the embodiments. No language in the specification should be construed as indicating any unclaimed element as essential to the practice of the embodiments.

In the following description, it is understood that terms such as "first," "second," and the like, are words of convenience and are not to be construed as limiting terms.

As used herein, unless otherwise indicated or made clear from the context, the term "physician" should be understood to include a surgeon or other interventional specialist preparing for and/or performing any one or more of the medical procedures described herein and, more broadly, should be understood to include any medical personnel, such as nurses, assisting a such surgeon or interventional specialist in preparing for or performing any one or more of the medical procedures described herein. Further, as used herein, the term "patient" shall be understood to include any type of mammal, including a human, on which a medical procedure such as, but not limited to a thrombectomy can be performed.

In the disclosure that follows, systems, catheters, and methods are described with respect to navigating tortuous neurovasculature (e.g., proximal internal carotid artery to the brain) to a treatment site and, at the treatment site, using a combination of aspiration and liquid injection to remove a blockage causing an acute ischemic stroke in a patient. It shall be understood that this is for the sake of clear and efficient description of various aspects and/or advantages of the systems, catheters, and methods described herein. In particular, as shall be appreciated in the description that follows, the context of treating acute ischemic stroke is useful for discussing the systems, catheters, and methods described herein, particularly with respect to advantages that may be broadly characterized into two categories that promote more efficient removal of blockages - namely, advantages related to size and shape of the catheter and advantages related decoupling size and shape from aspects of flexibility and strength of the catheter. While these two categories of advantages are not necessarily exclusive from one another - and, indeed, are realizable together in many of the examples described herein - the catheter features related to these two categories of advantages are generally discussed separately for the sake of clarity and to present aspects of the systems, catheters, and methods with appropriate scope. Thus, to orient the reader, it shall be understood that the description that follows focuses first on aspects of the size and shape of catheters that contribute to efficient removal of blockages and then focuses on aspects of catheters that facilitates decoupling flexibility and strength of a catheter from the size and shape of the catheter, which may have additional or alternative benefits for efficiently removing blockages.

While the present disclosure focuses on treatment of acute ischemic stroke, it shall be more generally understood that any one or more of the various, different systems, catheters, and/or methods described herein may be used to carry out thrombus removal in any one or more locations in veins and/or arteries of a patient, unless otherwise specified or made clear from the context. Further, the terms "thrombus," "blockage," "clot," and "embolus" shall be understood to be interchangeable with one another and shall not be interpreted to limit the types of treatments that may be carried out by the systems, catheters, and methods described herein. Thus, by way of example and not limitation, systems, catheters, and methods described herein may also, or instead, be used to remove thrombus from the peripheral vasculature of a patient (e.g., with catheter dimensions increased - such as doubled - for use in the peripheral vasculature). Still further, as used herein, the term "micro-guidewire" shall be understood to include any guidewire having a nominal outer diameter of less than or equal to 0.41 mm (e.g., 0.016 inch, 0.014 inch, 0.012 inch, 0.010 inch, 0.008 inch, and 0.007 inch guidewire sizes) or any other size as may be suitable for use in guiding any one or more of the various different catheters herein through neurovasculature to carry out treatment at a treatment site.

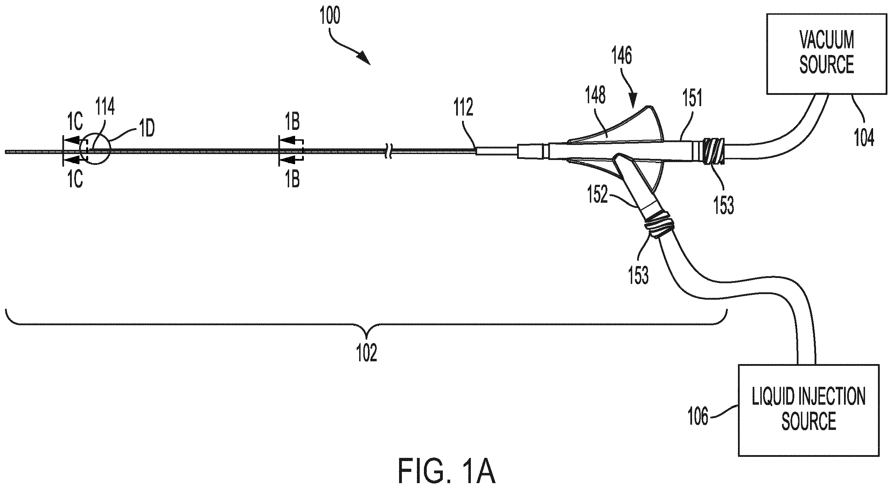

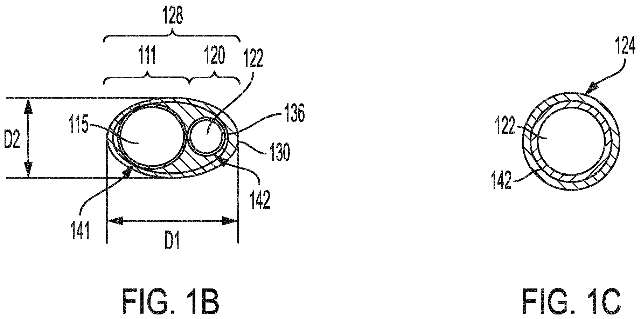

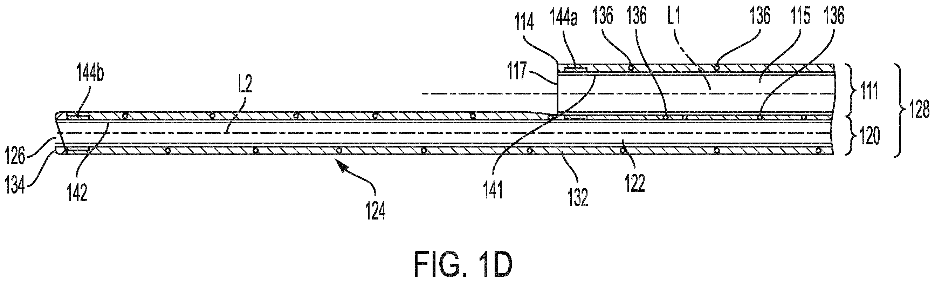

Referring now to FIGS. 1A-1D, a system 100 for removal of an occlusion within a vessel (e.g., removal of thrombus or other emboli causing an ischemic stroke) may include a double flow catheter 102, a vacuum source 104, and a liquid injection source 106 under controlled pressure. The double flow catheter 102 may include a first tube 111 having a proximal portion 112 and a distal portion 114 and defining a first lumen 115 therebetween. The distal portion 114 of the first tube 111 may define a first orifice 117 in fluid communication with the first lumen 115. Additionally, or alternatively, the double flow catheter 102 may include a second tube 120 defining a second lumen 122 parallel to the first lumen 115. The second tube 120 may include a nose 124 defining a portion of the second lumen 122 distal to the first lumen 115 and defining a second orifice 126. The first lumen 115 may have a first radial cross-sectional area greater than a second radial cross-sectional area of the second lumen 122. As described in greater detail below, the first lumen 115 having the first radial cross-sectional area greater than the second radial cross-sectional area of the second lumen 122 may facilitate achieving faster resolution of a blockage causing an acute ischemic stroke in a patient. For example, within an overall size profile that is navigable within the neurovasculature of a patient, the relatively small size of the second radial cross-sectional area of the second lumen 122 may accommodate a micro-guidewire with only a small amount of clearance while the relatively large size of the first radial cross-sectional area of the first lumen 115 may accommodate a high flow rate for aspiration and receiving the blockage and/or delivery of certain devices (e.g., a stent retriever), as needed, through the first lumen 115. Stated differently, the second radial cross-sectional area of the second lumen 122 may facilitate rapid and accurate navigation of the nose 124 of the double flow catheter 102 of the micro-guidewire to a treatment site (e.g., with the nose 124 decreasing the likelihood of the nose 124 inadvertently entering perpendicular artery ostium and becoming stuck, as described below) in the neurovasculature of a patient while the first radial cross-sectional area of the first lumen 115 may be useful for receiving the suctioned blockage and also rapidly delivering one or more devices to the treatment site without removing the double flow catheter 102, thus saving critical time as compared to treatments performed by withdrawing and introducing devices in series.

In use, as also described in greater detail below, the first lumen 115 of the double flow catheter 102 may be in fluid communication with the vacuum source 104, and the second lumen 122 of the double flow catheter 102 may be in fluid communication with the liquid injection source 106 under controlled pressure. At the treatment site , the first orifice 117 of the first tube 111 may be positioned proximal to a blockage in a vessel while the second orifice 126 may be positioned distal to the blockage in the vessel. As suction from the vacuum source 104 is directed to the blockage via the first orifice 117, pressurized liquid (e.g., saline) from the liquid injection source 106 under controlled pressure may be injected distally to the blockage in the vessel via the second orifice 126 to achieve pressure compensation in the vessel. Such pressure compensation may facilitate maintaining normal pressure in downstream vessels (e.g., downstream cerebral arteries, in the case of treating acute ischemic stroke) as occlusive material is displaced from the vessel. As compared to the use of a single-lumen aspiration catheter, the coordination of suction and pressurization in the vessel using the double flow catheter 102 may reduce the likelihood of unintentionally collapsing the vessel - thus, reducing the potential for brain injury - during treatment to remove the blockage, even as stronger and more effective suction is used. More specifically, as compared to use of a single-lumen aspiration catheter having the largest size that may be accommodated by a vessel (and, thus, in contact with the vessel such that no fluid may flow) to facilitate transporting a blockage, the pressure compensation provided by the double flow catheter 102 may reduce the risk that applying suction will collapse the vessel downstream of the blockage and cause brain injury as a result of stopping blood flow. Further, as compared to the use of a single-lumen aspiration catheter, the double flow catheter 102 may facilitate injection of liquid with chemical protection for the brain and/or clot dissolution as suction is being applied to remove the blockage and/or may facilitate measuring blood pressure/flow distal to the blockage being removed.

In general, the first tube 111 and the second tube 120 may be coupled to one another (e.g., fluidically isolated from one another, as described in greater detail below) and collectively define a body 128 proximal to the nose 124. In particular, the first tube 111 and the second tube 120 may be coupled to one another in an orientation in which the first lumen 115 and the second lumen 122 are adjacent to one another. As used in this context, adjacency of the first lumen 115 and the second lumen 122 shall be understood to include an arrangement of the first tube 111 and the second tube 120 in which the first lumen 115 and the second lumen 122 share at least a portion of a single wall in the body 128. Thus, for example, the first lumen 115 and the second lumen 122 shall be understood to be adjacent to one another in the orientation shown in FIG. 1B. Other adjacent orientations are discussed below.

With the first tube 111 and the second tube 120 coupled to one another, the first lumen 115 may define a first longitudinal axis L1 and the second lumen 122 may define a second longitudinal axis L2 parallel to, and radially spaced from, the first longitudinal axis L1 at least from the proximal portion 112 of the first tube 111 to the distal portion 114 of the first tube 111, as may be useful for achieving tight dimensional control over the first tube 111 and the second tube 120 using cost-effective fabrication techniques, such as extrusion. For example, the first tube 111 and the second tube 120 may be formed separately, as described in greater detail below, and fused to one another from the proximal portion 112 to the distal portion 114 of the first tube 111 such that the body 128 formed by the first tube 111 and the second tube 120 is a unitary body (e.g., without air, a line, or delamination in material between the first tube 111 and the second tube 120) with the nose 124 of the second tube 120. This may facilitate moving the first tube 111 and the second tube 120 in concert with one another to navigate tortuous neurovasculature to arrive at a blockage at treatment site. Further, or instead, the first tube 111 and the second tube 120 formed as a unitary body may facilitate positioning the first orifice 117 of the first lumen 115 proximal to the blockage for aspiration while the second orifice 126 of the second lumen 122 is positioned distal to the blockage to maintain pressure in the vessel as aspiration is applied. Continuing with this example, with the first tube 111 and the second tube 120 coupled to one another to form the body 128, the second lumen 122 may be fluidically isolated from the first lumen 115 such that the first lumen 115 and the second lumen 122 may be used carry out aspects of treatment (e.g., aspiration and liquid injection) independently of one another according to any one or more of the various, different techniques described herein.

The body 128 may have an outer surface 130 continuous from the proximal portion 112 to the distal portion 114 of the first tube 111. Among other advantages, the continuity of the outer surface 130 may facilitate effectively coating the outer surface 130 with one or more materials useful for advancing the first tube 111 and the second tube 120 through tortuous neurovasculature of the patient to position the first orifice 117 of the first tube 111 and the second orifice 126 of the second tube 120 relative to the blockage in the vessel. For example, the outer surface 130 of the body 128 may include a hydrophilic coating (e.g., hydrophilic material grafted onto the outer surface 130) to reduce friction of the outer surface 130 moving through a vessel. That is, given its affinity for water, the hydrophilic coating may secure water on the outer surface 130 to form a water/vessel interface as the outer surface 130 moves along a vessel. Such a water/vessel interface has lower friction than a polymer/vessel interface that would exist in the absence of the hydrophilic coating. In certain implementations, an outer surface of the nose 124 may additionally, or alternatively, include a hydrophilic coating to reduce friction as nose 124 moves through a vessel, toward a treatment site.

The dimensions of the outer surface 130 represent boundary conditions at least related to the smallest size vessel through which the first tube 111 and the second tube 120 may be navigated, sizing the first lumen 115 and the second lumen 122 relative to one another, and wall sizes that support flexibility for navigating tortuous vasculature while also withstanding forces associated with aspiration through the first lumen 115 and liquid injection through the second lumen 122. For example, in instances in which the double flow catheter 102 is used for treatment in neurovasculature of a patient, the outer surface 130 of the body 128 may have a maximum radial dimension greater than about 1 mm and less than about 6 mm. Such dimensions may be close to - but slightly smaller than - the dimensions of cerebral arteries such that outer surface 130 of the body 128 may move through the neurovasculature with little or no damage to vessels as treatment is carried out. As an example, for removal of a clot in the M1 section of a cerebral artery, the maximum radial dimension of the outer surface 130 of the body 128 may be greater than about 2 mm and less than about 2.5 mm. As another example, for removal of a clot in the M2 section of a cerebral artery, the maximum radial dimension of the outer surface 130 of the body 128 may be greater than about 1.5 mm and less than about 2 mm. As yet another example, for removal of a clot in the M3 section of a cerebral artery, the maximum radial dimension of the outer surface 130 of the body 128 may be greater than about 1 mm and less than about 1.5 mm. As still another example, for removal of a clot in the C1 section of an internal carotid artery, the maximum radial dimension of the outer surface 130 of the body 128 may be greater than about 2.5 mm and less than about 3.2 mm. As another example, for removal of a clot in the external carotid artery or common carotid artery, the maximum radial dimension of the outer surface 130 of the body 128 may be greater than about 3 mm and less than about 6 mm. As yet another example, for removal of a clot in the basilar artery, the maximum radial dimension of the outer surface 130 of the body 128 may be greater than about 2.5 mm and less than about 3.5 mm. As still another example, for removal of a clot in the P1 section of a posterior cerebral artery, the maximum radial dimension of the outer surface 130 of the body may be greater than 2 mm and less than about 2.5 mm. Other size ranges may be used for other types of treatment (e.g., larger size ranges may be used in implementations in which the system 100 to remove blockages in the peripheral vasculature of a subject).

The circumference of the outer surface 130 of the body 128 may be generally any continuous curvilinear shape along an axial length of the body 128 from the proximal portion 112 to the distal portion 114 of the first tube 111, with one or more rounded edges useful for making sliding contact with the vessel with little or no damage to the vessel as the double flow catheter 102 is moved to the treatment site. For example, the circumference of the outer surface 130 of the body 128 may have an oblong shape with a major dimension D1 and a minor dimension D2, where the major dimension D1 is greater than the minor dimension D2 (e.g., an oval shape or a stadium-shape). As a specific example, with less material requiring bending, the body 128 may be more flexible in a first plane defined by the minor dimension D2 and the first longitudinal axis L1 of the oblong shape than in a second plane defined by the major dimension D1 and the first longitudinal axis L1 of the oblong shape. This may be particularly useful as a self-aligning feature of the double flow catheter 102 to facilitate moving the major dimension D1 of the oblong shape through a vessel as the double flow catheter 102 is navigated through tortuous vasculature while providing larger overall cross-sectional area - as compared to a circular cross-section - for accommodating the first lumen 115 and the second lumen 122. Stated differently, as the body 128 is navigated through tortuous vasculature, forces on the outer surface 130 of the body 128 may position the outer surface 130 of the body 128 in an orientation of least resistance - namely, an orientation of bending about an axis defined by the major dimension D1 of the oblong shape - to facilitate moving the body 128 through the vasculature.

In some implementations, the shape of the circumference of the nose 124 may differ from the shape of the circumference of the outer surface 130 of the body 128. For example, while the circumference of the outer surface 130 of the body 128 may be oblong to facilitate self-alignment as the body 128 moves through tortuous neurovasculature, the circumference of the nose 124 may be substantially circular (allowing for small variations within manufacturing tolerance). Among other things, the substantially circular circumference of the nose 124 may be useful for fabricating the second tube 120 cost-effectively as an extrusion having a nominally uniform wall thickness and a substantially circular circumference along the length of the second tube 120 prior to coupling the second tube 120 to the first tube 111 to form the body 128.

In general, the shape of the first radial cross-section of the first lumen 115 and the shape of the second radial cross-section of the second lumen 122 may be any one or more of various shapes useful for accommodating both the first radial cross-sectional area and the second radial cross-sectional area within a boundary defined by the outer surface 130 of the body 128. The shape of the first radial cross-sectional area of the first lumen 115 may be substantially constant, along the first lumen 115, from the proximal portion 112 to the first orifice 117 defined by the distal portion 114 of the first tube 111. In some instances, the shape of the second radial cross-sectional area of the second lumen 122 may be substantially constant along the entire longitudinal dimension of the second lumen 122. It shall be appreciated that the substantially constant shape of the first radial cross-sectional area of the first lumen 115 and/or the second radial cross-sectional area of the second lumen 122 may facilitate, among other things, achieving tight dimensional tolerance along the first lumen 115 and/or the second lumen 122. It shall be further appreciated that, in this context, the substantially constant shape of the first radial cross-sectional area of the first lumen 115 and/or the second radial cross-sectional area of the second lumen 122 may allow for small variations (within ± 5 percent) from an ideal constant shape, such as variations associated with manufacturing tolerances in extrusion or other fabrication techniques used to form the first tube 111 and/or the second tube 120.

In some instances, at least one of the first radial cross-section of the first lumen 115 or the second radial cross-section of the second lumen 122 may include a respective arcuate segment. For example, in some instances, the first radial cross-sectional area and the second radial cross-sectional area may each have a respective rounded shape (e.g., a shape without convergence of straight edges). Such rounded shapes may, among other things, reduce the likelihood of unintended force concentrations - thus, reducing the likelihood of kinking - while making efficient use of available cross-sectional area within the boundary defined by the outer surface 130 of the body 128. For example, the first radial cross-section of the first lumen 115 may be elliptic (e.g., circular or oval) from the proximal portion 112 to the distal portion 114 of the first tube 111. Additionally, or alternatively, the second radial cross-section of the second lumen 122 may be elliptic (e.g., circular or oval) along a longitudinal dimension of the second lumen 122.

In general, the first radial cross-sectional area of the first lumen 115 and the second radial cross-sectional area of the second lumen 122 may be sized relative to one another to accommodate the primary function of each lumen. That is, in the context of treating acute ischemic stroke, the first radial cross-sectional area of the first lumen 115 may be sized to accommodate receiving the blockage suctioned into and through the first lumen 115 with efficient use of suction at the treatment site while also accommodating one or more types of other devices (e.g., a stent retriever) that may be used, as necessary or desirable, to assist in removing the blockage. Thus, while it may be generally desirable for the first radial cross-sectional area to be as large as possible, the overall cross-sectional area defined by the outer surface 130 of the body 128 and accommodation of size requirements of the second radial cross-sectional area may, among other considerations, serve as boundary conditions on the maximum allowable size of the first radial cross-sectional area. For example, as also mentioned above, the second radial cross-sectional area of the second lumen 122 may be sized to follow a micro-guidewire closely (e.g., with only a small amount of clearance between the second lumen 122 and the micro-guidewire) for accurate navigation of the body 128 of the double flow catheter 102 while also having an area large enough for delivery of certain devices such as a stentriever or for liquid injection (e.g., injection of contrast media through the second lumen 122 ) using hand pumping or other low-cost and ubiquitous equipment. Thus, at least from the proximal portion 112 to the distal portion 114 of the first tube 111, a ratio of the first radial cross-sectional area to the second radial cross-sectional area may be greater than about 3:1 and less than about 11:1. As an example, the first radial cross-sectional area may be greater than 1 mm² and less than 2.5 mm², and the second radial cross-sectional area may be greater than or equal to 0.1 mm² and less than or equal to 0.25 mm². Further, or instead, a maximum radial dimension of the first lumen 115 having the first radial cross-sectional area may be greater than about 1 mm and less than about 2.0 mm. A maximum radial inner dimension of the second lumen 122 may be greater than about 0.2 mm and less than about 0.6 mm (including, for example, along the portion of the second lumen 122 defined by the nose 124) in some implementations. As a specific example, at least along the portion of the second lumen 122 defined by the nose 124, the maximum radial dimension of the second lumen 122 may be within the range of 0.41 mm to 0.43 mm to facilitate following a micro-guidewire according to the various different techniques described herein.

With maximum radial dimensions and ranges of radial cross-sectional areas of the first lumen 115 and the second lumen 122 at least partially dictated by the foregoing considerations related to aspiration and injection at the treatment site, other dimensional parameters may be a function of the treatment to be carried out. Generally, for treatment of acute ischemic stroke, the longitudinal dimension of the body 128 from the proximal portion 112 to the distal portion 114 of the first tube 111 may be greater than about 1 m and less than about 1.7 m to facilitate reaching various portions of the cerebral vasculature. The nose 124 of the second tube 120 may include a proximal section 132 and a distal section 134, with the proximal section 132 adjacent to the distal portion 114 of the first tube 111 and the distal section 134 of the nose 124 distally away from the distal portion 114 of the first tube 111. From the proximal section 132 of the nose 124 to the distal section 134 of the nose 124, a maximum outer dimension of the nose 124 may be greater than about 0.4 mm and less than about 1.0 mm. Further, or instead, from the proximal section 132 to the distal section 134, the nose 124 an outer circumference of the nose 124 may be circular, such as in instances in which the second tube 120 initially has a circular outer circumference before being coupled with the first tube 111 to form the body 128.

The length of the nose 124 shall be understood to be the axial dimension from the proximal section 132 of the nose 124 to the second orifice 126 defined by the distal section 134 of the nose 124. As an example, the length of the nose 124 may be greater than about 15 mm and less than about 75mm from the proximal section 132 to the distal section 134 of the nose 124. In certain implementations, the length of the nose may be selected to be longer than the clot to be removed, and the maximum radial dimension of the outer surface 130 of the body 128 may be selected to be the largest possible size to fit in the target artery.

In general, the nose 124 of the double flow catheter 102 may have a smaller radial dimension than the body 128 such that the nose 124 is more flexible than the body 128 without kinking. As compared to a single-lumen catheter or to a catheter with co-terminal tubes at a distal end of the catheter, the small radial dimension of the nose 124 of the double flow catheter 102 may improve navigation in the vicinity of an ostium as the catheter 102 is moved through a vessel. More specifically, the nose 124 of the double-flow catheter 102 may reduce the likelihood of the double-flow catheter 102 entering the wrong vessel. That is, as the nose 124 is moved over a micro-guidewire in the vessel, the flexibility of the nose 124 may facilitate correctly navigating past the ostium while remaining in the vessel. As shall be readily appreciated, once the nose 124 is past the ostium, the body 128 - which is stiffer than the nose 124 - follows the nose 124 along the correct path past the ostium. Thus, as may be appreciated from the foregoing, the nose 124 may reduce the time of intervention by reducing the potential for entering the wrong vessel as the nose 124 moves past an ostium. This is particularly advantageous in implementations in which the catheter 102 is navigated in body vasculature in which an artery ostium is near a curve, such where the ophthalmic artery branches from the internal carotid artery.

Example size configurations for treatment of certain types of cerebral blockages are as follows:
- For removal of a clot in the M1 section of a cerebral artery, the length of the nose 124 may be in the range of about 20 mm to about 35 mm, the maximum radial dimension of the outer surface 130 of the body 128 may be in the range of about 2 mm to about 2.5 mm, and the second radial cross-sectional area of the second lumen 122 at the second orifice 126 may be in the range of about 0.1 mm² to about 0.25 mm².
- For removal of a clot in the M2 section of a cerebral artery, the length of the nose 124 may be in the range of about 20 mm to about 35 mm, the maximum radial dimension of the outer surface 130 of the body 128 may be in the range of about 1.5 mm to about 2 mm, and the second radial cross-sectional area of the second lumen 122 at the second orifice 126 may be in the range of about 0.1 mm² to about 0.2 mm².
- For removal of a clot in the M3 section of a cerebral artery, the length of the nose 124 may be in the range of about 15 mm to about 35 mm, the maximum radial dimension of the outer surface 130 of the body 128 may be in the range of about 0.2 mm to about 0.5 mm, and the second radial cross-sectional area of the second lumen 122 at the second orifice 126 may be in the range of 0.05 mm² to 0.1 mm².
- For removal of a clot in the C1 section of an internal carotid artery, the length of the nose 124 may be in the range of about 25 mm to about 40 mm, the maximum radial dimension of the outer surface 130 of the body 128 may be in the range of about 2.5 mm to about 4 mm, and the second radial cross-sectional area of the second lumen 122 at the second orifice 126 may be in the range of about 0.1 mm² to about 0.25 mm².
- For removal of a clot in the external carotid artery or common carotid artery, the length of the nose 124 may be in the range of about 30 mm to about 50 mm, the maximum radial dimension of the outer surface 130 of the body 128 may be in the range of about 4 mm to about 15 mm, and the second radial cross-sectional area of the second lumen 122 at the second orifice 126 may be in the range of 0.25 mm² to 1 mm².
- For removal of a clot in the basilar artery, the length of the nose 124 may be in the range of about 25 mm to about 40 mm, the maximum radial dimension of the outer surface 130 of the body 128 may be in the range of about 2.5 mm to about 5 mm, and the second radial cross-sectional area of the second lumen 122 at the second orifice 126 may be in the range of 0.1 mm² to 0.25 mm².
- For removal of a clot in the P1 section of a posterior cerebral artery, the length of the nose 124 may be in the range of about 20 mm to about 35 mm, the maximum radial dimension of the outer surface 130 of the body 128 may be in the range of about 2 mm to about 2.5 mm, and the second radial cross-sectional area of the second lumen 122 at the second orifice 126 may be in the range of 0.1 mm² to 0.25 mm².

Having described certain aspects of size and shape of the double flow catheter 102, attention is turned now to discussion of aspects of the physical properties of features of the double flow catheter 102 that may advantageously decouple certain aspects of size and shape from flexibility and strength to address challenges associated with balancing anatomic constraints against rapid, safe, and robust removal of blockage, with such challenges being particularly prevalent in removing blockages for treatment of acute ischemic stroke. In particular, in the discussion that follows, features of the double flow catheter 102 that may be useful for decoupling size and shape from flexibility and strength are discussed in the context of an axial stiffness profile of the body 128 and the nose 124 that achieves performance criteria of strength, kink resistance, pushability, and navigability of the double flow catheter 102, for example, for use in removing blockages to treat acute ischemic stroke.

While stiffness may be generally useful for kink resistance, pushability, and strength, the double flow catheter 102 must also nevertheless be flexible enough to navigate tortuosity of the neurovasculature for placement at the treatment site. Accordingly, to balance these competing considerations, the body 128 may have an axial stiffness profile characterized by an overall bend radius that decreases from the proximal portion 112 to the distal portion 114 of the first lumen 115. As used herein, bend radius (and variants thereof) shall be understood to refer to the minimum radius (corresponding to inside curvature) one may bend a given tube without kinking. Thus, the overall bend radius of the body 128 shall be understood to refer to the minimum radius one may bend the first lumen 115 and the second lumen 122 - with the first lumen 115 and the second lumen 122 coupled to one another to form the body 128 - without kinking either of the first lumen 115 or the second lumen 122. In certain instances, the overall bend radius of the body 128 may decrease in discrete sections (e.g., as step changes from section to section) along at least a portion of a longitudinal dimension of the body 128 and/or the nose 124. Additionally, or alternatively, the overall bend radius of the body 128 may decrease according to a gradient (e.g., according to a predetermined profile) along at least a portion of a longitudinal dimension of the body 128 and/or the nose 124.

In general, the first tube 111 and the second tube 120 may each have respective physical properties that are independent of one another. These differences in physical properties may be useful for, among other things, achieving an overall physical property of the body 128 while also accommodating the different performance criteria associated with different primary uses and/or secondary uses of the first tube 111 and the second tube 120 during treatment. As a specific example, the first tube 111 may have a radial compressive strength of at least about 1050 mmHg along the first lumen 115 to resist collapsing as 760 mmHg of vacuum pressure is applied in the first lumen 115 and liquid moving through the second lumen 122 has a dynamic pressure of about 300 mmHg for injection into the vessel. Similarly, the second tube 120 may have a burst pressure (a radial tensile strength) of at least about 1050 mmHg along at least a portion of the second lumen 122 proximal to the nose 124 to resist tearing as 760 mmHg of vacuum pressure is applied in the first lumen 115 and liquid moving through the second lumen 122 has a dynamic pressure of about 300 mmHg for injection into the vessel. It shall be appreciated that the foregoing values of radial compressive strength and burst pressure represent minimum values and that these values may be significantly higher in many implementations. For example, to accommodate injection of contrast media through one or both of the first lumen 115 or the second lumen 122 (e.g., for visualization of the blockage at the treatment site using medical imaging), the first tube 111 and/or the second tube 120, as the case may be, may have a burst pressure greater than about 37500 mmHg.

Apart from differences in physical properties attributable to differences in geometry between the first tube 111 and the second tube 120, the physical properties of the first tube 111 and/or the second tube 120 may be tuned (e.g., relative to one another and with respect to overall performance of the double flow catheter 102) by varying material composition of the first tube 111 and/or the second tube 120 as described in the following paragraphs.

In general, the first tube 111 and the second tube 120 may each be at least partially formed of one or more polymers (e.g., polyurethane, silicone, polyamide, or any of various other biocompatible polymers). As described in greater detail below, the type and/or properties of polymers used in each of the first tube 111 and the second tube 120 may vary along a longitudinal dimension of each respective tube to form the body 128 with progressively increasing flexibility in a direction defined from the proximal end portion 112 to the distal end portion 114 of the first tube 111, with the nose 124 of the second tube 120 being the most flexible portion of the catheter 102 in some instances. Such longitudinal variation of polymeric composition of each tube shall be referred to herein as a "polymeric composition profile." Unless otherwise specified or made clear from the context, the respective polymeric composition profile of a given tube may be formed by a plurality of discrete sections (e.g., 6 or more sections), with each discrete section having a substantially uniform polymeric composition (allowing for small variations in composition with design tolerance). Continuing with this example, the plurality of discrete sections of a tube may be arranged relative to one another in order of decreasing Shore D hardness to form the polymeric composition profile of a given tube. For example, a polymeric composition profile of a tube may be formed by discrete sections coupled to one another along an interface perpendicular to a longitudinal axis of the tube. Further or instead, a polymeric composition profile of a tube may be formed by discrete sections coupled to one another at an interface oblique relative to the longitudinal axis such that the transition in softness between adjacent sections is gradual along the longitudinal axis.

In certain implementations, the first tube 111 may be formed of a first polymeric composition profile varying in composition at least in an axial direction along at least a portion of the first tube 111. Additionally, or alternatively, the second tube 120 may be formed of a second polymeric composition profile varying in composition at least in an axial direction along at least a portion of the second tube 120.

In some implementations, the first polymeric composition profile and/or the second polymeric composition profile may have softness that varies along the longitudinal dimension of the body 128 and/or the length of the nose 124. As an example, the first polymeric composition profile and/or the second polymeric composition profile may have softness that varies within a predetermined range (e.g., within the range of 1 Shore D to 100 Shore D, such as 30 Shore D to 40 Shore D) along the longitudinal dimension of the body 128 and/or the length of the nose 124. The first polymeric composition profile may be progressively softer in a distal direction parallel to the first longitudinal axis L1 of the first tube 111 and further, or instead, the second polymeric composition profile may be progressively softer in a distal direction parallel to the second longitudinal axis L2 of the second tube 120. In instances in which one or both of the first polymeric composition profile or the second polymeric composition profile is progressively softer in a distal direction, the overall softness of the body 128 may increase progressively in the distal direction, as may be observable as a progressively decreasing overall bend radius of the body 128 in the distal direction.

The first polymeric composition profile may be different from the second polymeric composition profile at certain axial positions along the longitudinal dimension of the body 128 to accommodate different performance requirements of the first tube 111 relative to those of the second tube 120 while also achieving performance requirements of the first tube 111 and the second tube 120 coupled to one another to form the body 128. However, even in instances in which the first polymeric composition profile and the second polymeric composition profile differ from one another, the first polymeric composition profile and the second polymeric composition profile may have the same polymer composition in at least one axial position along the longitudinal dimension of the body 128 from the proximal portion 112 to the distal portion 114 of the first tube 111. For example, in instances in which the first polymeric composition profile includes discrete sections of different polymers and the second polymeric composition profile includes discrete sections of different polymers to impart progressive softness to the body 128, at least one discrete section of the first polymeric composition profile may be the same as a corresponding discrete section of the second polymeric composition profile at a given axial position along the longitudinal dimension of the body 128 such that a single polymeric composition circumscribes each of the first lumen 115 and the second lumen 122 and forms the outer surface 130 as the given axial position.

In some implementations, it may be useful for the first polymeric composition profile and the second polymeric composition profile to have the same polymer composition at either longitudinal end portion of the body 128 - corresponding to an axial position along the proximal portion 112 and/or the distal portion 114 of the first tube 111 - where it may be desirable for the first polymeric composition profile and the second polymeric composition profile to each include a polymer at one or both extreme ends of a range of hardness. That is, returning to the example in which the first polymeric composition profile and/or the second polymeric composition profile may have softness within a range of 1 Shore D to 100 Shore D (e.g., 30 Shore D to 40 Shore D), it may be useful for the first polymeric composition profile and/or the second polymeric composition profile to be formed of the same polymer having 30 Shore D hardness at an axial position corresponding to the proximal portion 112 of the first tube 111, as may be useful for imparting pushability at that axial position. Continuing with this example, it may be further, or alternatively, useful for the first polymeric composition profile and/or the second polymeric composition profile to be formed of the same polymer having 40 Shore D hardness at an axial position corresponding to the distal portion 114 of the first tube 111, as may be useful for achieving the smallest overall bend radius achievable with polymers within the range of hardness.

The second polymeric composition profile of the second tube 120 may be softest along at least a portion of the nose 124 between the proximal section 132 and the distal section 134 of the nose 124 and, in some instances, the second polymeric composition profile may be constant in an axial direction from the proximal section to the distal section of the nose. As compared to the use of harder polymers, the softness of the second polymeric composition profile along the nose 124 may advantageously reduce the likelihood of unintended damage to tissue that comes into contact with the nose 124 as the nose 124 is moved along a micro-guidewire to navigate tortuosity of the neurovasculature and/or as the nose 124 follows the micro-guidewire to move between the blockage and the vessel wall and cross the blockage such that the second orifice 126 distal to the blockage to carry out treatment according to any one or more of the various different techniques described herein. Further, or instead, the second polymeric composition profile of the second tube 120 being softest along the nose 124 may facilitate moving the nose 124 efficiently - with little or no need for repeated distal and proximal movement of the double flow catheter 102 and without the need for specifically dedicated navigation features such as a bulb - through tortuous bifurcations of vessels, such as bifurcation of an ophthalmic artery from the internal carotid artery and/or bifurcation of the middle cerebral artery and the anterior cerebral artery. That is, as compared to catheters formed of harder material, the softness of the nose 124 may increase the likelihood that, as the nose 124 follows a micro-guidewire, the nose 124 may move toward a centerline of an artery in response to making inadvertent contact with a wall of a substantially perpendicular artery. Further, or instead, the nose 124 may include a blunt end in some instances. Bluntness of the nose 124, alone or in combination with softness of the nose 124, may facilitate redirecting the nose 124 back toward a centerline of the vessel following contact with tissue of the vessel with little or no harm to tissue of the vessel. As compared to shapes with sharp edges, bluntness of the nose 124 may reduce the likelihood of damaging the vessel wall as the nose 124 crosses the blockage by following the micro-guidewire to between the blockage and the vessel wall.

While composition of the first polymeric composition profile and the second polymeric composition profile may be a function of one or more performance parameters associated with aspiration through the first lumen 115 and/or liquid injection through the second lumen 122, it shall be appreciated that respective compositions of first polymeric composition profile and the second polymeric composition profile may also be selected based on manufacturing techniques used to couple the first tube 111 and the second tube 120 to one another to form the body 128 of the double flow catheter 102. For example, the first polymeric composition profile and the second polymeric composition profile may have the same, or at least similar, melting points at each axial position along the longitudinal dimension of the body 128 to facilitate coupling the first tube 111 and the second tube 120 to one another via fusing. That is, the first tube 111 and the second tube 120 may each be heated to the melting point of one or both of the first polymeric composition profile and/or the second polymeric composition profile with the first tube 111 and the second tube 120 in contact with one another from the proximal portion 112 to the distal portion 114 of the first tube 111 and with the first longitudinal axis L1 and the second longitudinal axis L2 parallel to one another. Continuing with this example, as the first polymeric composition profile of the first tube 111 and the second polymeric composition profile of the second tube 120, the first tube 111 and the second tube 120 become fused together from the proximal portion 112 to the distal portion 114 of the first tube 111 to form the body 128. Additionally, or alternatively, the first polymeric composition profile of the first tube 111 and the second polymeric composition profile blend of the second tube 120 may be selected for coextrusion or other approaches that may be used for coupling the first tube 111 and the second tube 120 to one another to form the body 128.

In some implementations, the body 128 may include one or more reinforcement materials 136 (hereinafter referred to as the reinforcement material 136 for clarity and efficiency of description) at least along the longitudinal dimension of the body 128 from the proximal portion 112 to the distal portion 114 of the first tube 111. The reinforcement material 136 may include any bio-compatible material that is discrete from and embedded in the first polymeric composition profile of the first tube 111 and/or in the second polymeric composition profile of the second tube 120. Examples of such material include: stainless steel, nitinol, tungsten, polymeric fibers, or a combination thereof.

The reinforcement material 136 may increase local strength (e.g., increase radial compressive strength, increase burst pressure, increase resistance to elongation, increases resistance to kinking, reduces likelihood of collapse under vacuum pressure, or any combination thereof) of the first tube 111, the second tube 120, or both than is otherwise achievable with the first polymeric composition profile and the second polymeric composition profile alone. Stated differently, the longitudinal variations in one or more physical properties of the first tube 111, the second tube 120, or both may be achieved by varying the amount and/or type of the reinforcement material 136 along at least the longitudinal dimension of the body 128 and, in some cases, along the length of the nose 124. As an example, density of one or more reinforcement materials per unit of axial length of the body 128 may decrease in an axial direction from the proximal portion 112 to the distal portion 114 of the first tube 111, as may be useful for achieving high stiffness along an axial position of the body 128 corresponding to the proximal portion 112 of the first tube 111 (e.g., to enhance pushability of the body 128) while achieving high flexibility along an axial position of the body 128 corresponding to the distal portion 114 of the first tube 111 (e.g., to enhance navigability of the body 128 through tortuous neurovasculature).

The reinforcement material 136 may circumscribe at least one of the first lumen 115 or the second lumen 122. For example, the reinforcement material 136 may circumscribe the first lumen 115 and the second lumen 122 collectively such that the reinforcement material 136 is embedded in the body 128 between the outer surface 130 of the body and each of the first lumen 115 and the second lumen 122, as may be useful for imparting overall strength to the body 128. As another example, the reinforcement material 136 may circumscribe at least one of the first lumen 115 or the second lumen 122 individually such that the reinforcement material 136 is embedded in the body 128 between the first lumen 115 and the second lumen 122, as may be useful for imparting different strength characteristics to the first tube 111 and/or the second tube 120 while achieving overall physical properties of the body 128.

In some implementations, the reinforcement material 136 may include one or more wires which, in general, may be wrapped about the body 128 and/or individually about each one of the first tube 111 and/or the second tube 122 according to any one or more patterns as may be useful for imparting local strength and, in some cases, a strength gradient along at least the longitudinal dimension of the body 128 from the proximal portion 112 to the distal portion 114 of the first tube 111. As an example, the reinforcement material 136 may include one or more wires wrapped in a pattern with a decreasing pitch (e.g., as with a coil pattern and/or a braid pattern), having decreasing cross-sectional area, having different composition, or a combination thereof along the body 128 in the axial direction from the proximal portion 112 to the distal portion 114 of the first tube 111. Additionally, or alternatively, in instances in which the reinforcement material includes one or more wires, these one or more wires may form a coil, an overlapping and/or interwoven pattern (e.g., braiding), or a combination thereof about at last one of the first tube 111, the second tube 120, or both. For example, the first tube 111 may have braiding along the entire length of the first tube 111 from the proximal portion 112 to the distal portion 114, as may be useful for reducing the likelihood of collapse and/or kinking of the first tube 111 as the first tube 111 is bent and the first lumen 115 is under vacuum pressure. Further, it shall be appreciated that each of the one or more wires may have any one or more of various different cross-sectional radial areas along the length of the respective wire to facilitate balancing competing considerations associated with providing reinforcement while accommodating overall flexibility of the body 128. Thus, for example, each of the one or more wires may have a radial cross-sectional area that is circular or rectangular along a longitudinal dimension of the respective wire. Still further, while each wire may be identical to each of the other wires in instances in which the reinforcement material 136 includes a plurality of wires, it shall be appreciated that the wires may have different sizes, shapes, and/or composition from one another, to the extent useful for achieving a predetermined flexibility profile of the body 128.

While the first lumen 115 and the second lumen 122 may be unlined in some implementations, the double flow catheter 102 may include any one or more linings inside the first lumen 115 and/or in the second lumen 122 to facilitate passing components through the first lumen 115 and/or the second lumen 122 during the course of treatment to remove a blockage. For example, the double flow catheter 102 may include a first lining 141 in the first lumen 115 and a second lining in the second lumen 122, and the first lining 141 and the second lining 142 may each be hydrophobic, although each lining may have a different composition in some instances. As a more specific example, at least one of the first lining 141 or the second lining 142 may include polytetrafluoroethylene (PTFE), a polymer having a lower surface energy than PTFE, or a combination thereof. In use, the hydrophobicity of the first lining 141 and the second lining 142 may reduce friction force that must be overcome to move components through the first lumen 115 and the second lumen 122 while providing good resistance to shedding (as compared to hydrophilic material), thus reducing the likelihood of unintended introduction of lining material into the body of the patient in instances in which it is useful or necessary to move a device through one or both of the first lumen 115 or the second lumen 122 during the course of treatment.

In some implementations, the double flow catheter 102 may include a first radiopaque marker 144a and a second radiopaque marker 144b. The first radiopaque marker 144a and the second radiopaque marker 144b may include rings, dots, or a combination thereof of radiopaque material (e.g., platinum, platinum iridium, tantalum gold, radiopaque polymer, or the like), as may be useful for identifying features of the nose 124 using medical imaging. The first radiopaque marker 144a and the second radiopaque marker 144b may be axially spaced relative to one another along the double flow catheter 102 to facilitate identifying the axial extent of the nose 124 relative to the blockage at the treatment site. As an example, the first radiopaque marker 144a may be disposed along the distal portion 114 of the first tube 111 to provide an indication of the position of the first orifice 117 of the first tube 111 relative to the blockage, and the second radiopaque marker may be disposed along the nose 124 (e.g., along the distal section 134 of the nose 124) to provide an indication of the position of the second orifice 126 relative to the blockage. Thus, continuing with this example, a physician may use medical imaging to identify the positions of the first radiopaque marker 144a and the second radiopaque marker 144b relative to the blockage in the vessel. In particular, the physician may inject contrast media through the first lumen 115 and the second lumen 122 and use imaging to confirm that the nose 124 is properly positioned for treatment of the blockage in the vessel by identifying the second radiopaque marker 144b as distal to the blockage while the first radiopaque marker 144a is proximal to the blockage.

In general, the first tube 111 and the second tube 120 may be connected in fluid communication with the vacuum source 104 and the liquid injection source 106 under controlled pressure according to any one or more of various different techniques that may be useful for a given medical procedure while allowing for manipulation of the body 128 of the double flow catheter 102 from outside of the patient. Thus, for some types of procedures (such as procedures associated with removing blockages from neurovasculature to treat acute ischemic stroke), the double flow catheter 102 catheter may include a hub 146 coupled to the body 128 along an axial position of the proximal portion of the first tube 111.

The hub 146 may include a housing 148, a first extension tube 151, a second extension tube 152, a first connector 153, and a second connector 154. The first lumen 115 of the first tube 111 of the body 128 may be releasably connectable in fluid communication with the vacuum source 104 via a first flow channel at least partially defined by the housing 148, the first extension tube 151, and the first connector 153. The second lumen 122 of the second tube 120 of the of the body 128 may be releasably connectable in fluid communication with the liquid injection source 106 under controlled pressure with or without lyse drug via a second flow channel, which is fluidically isolated from the first flow channel and at least partially defined by the housing 148, the second extension tube 152, and second connector 154. In use, the physician may manipulate the body 128 in the neurovasculature of the patient by moving the housing 148 outside of the body of the patient. Further, or instead, to facilitate achieving reliable fluid tight connections of the hub 146 and each of the vacuum source 104 and the liquid injection source 106 under controlled pressure, the first connector 153 and the second connector 154 may each be Luer lock fitting or another type of fitting that may be quickly connected and disconnected using simple manual motion (e.g., twisting).

In general, the vacuum source 104 may be any source of suction that may be generated outside of the body of the patient and delivered to the first orifice 117 of the first tube 111 (via the first lumen 115) with force that may dislodge the blockage from the vessel and move the blockage into the first lumen 115 for removal from the patient. Thus, for example, the vacuum source 104 may include a vacuum pump. Additionally, or alternatively, the vacuum source 104 may include a syringe (e.g., a 60 mL syringe) and suction may be applied by moving a plunger of the syringe away from an end-of-travel position in a barrel of the syringe.

The liquid injection source 106 under controlled pressure may generally include any source of liquid that may be pressurized outside of the body of the patient and delivered to the second orifice 126 of the second tube 120 (via the second lumen 122) at a pressure approximating normal anatomical pressure in the vessel (e.g., in the range of 250 mmHg to 300 mmHg) to reduce the likelihood of vessel collapse and facilitate moving the thrombus T with flow back toward the first orifice 117 while nevertheless remaining at a pressure that may be safely withstood by the vessel. Thus, in some instances, the liquid injection source 106 under controlled pressure may include a pump. Additionally, or alternatively, the liquid injection source 106 under controlled pressure may include a pouch, an IV bag, and a manometer. Continuing with this example, the pouch may be positioned about the IV bag, and the pouch may be inflated (e.g., through manual pumping) about the IV bag to move saline out of the IV bag at a pressure measured by the manometer. The operator may monitor pressure readings on the manometer to maintain the pressure of saline at less than about 300 mmHg such that the saline may be safely introduced into the vessel to support the vessel as suction is applied to the vessel via the vacuum source.

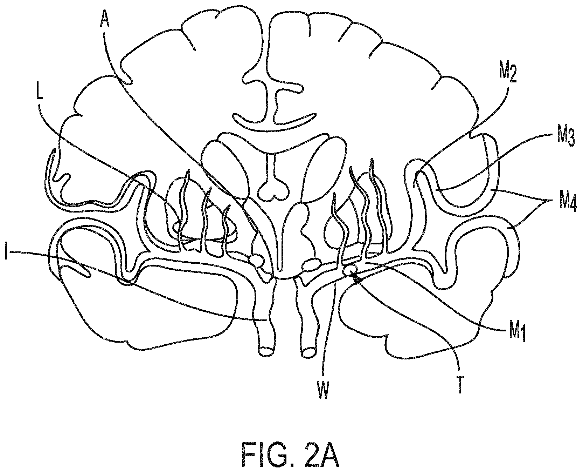

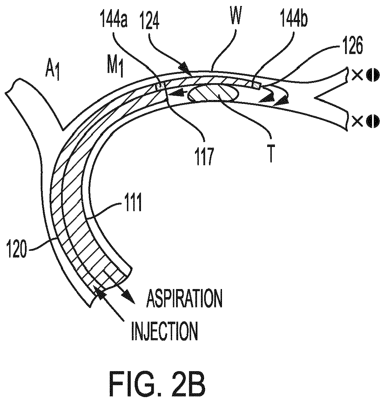

Referring now to FIGS. 1A-1D and FIGS. 2A-2B, having described various aspects of the system 100, attention is now directed to discussion of an exemplary method of using the system 100 to remove a thrombus T from neurovasculature of a patient suffering from an acute ischemic stroke. While the thrombus T is shown in a section of a middle cerebral artery, it shall be appreciated that this is by way of example and not limitation. Unless otherwise specified or made clear from the context, the system 100 may be used to remove blockages from any one or more positions in the neurovasculature described herein.

Prior to intervention with the system 100 to treat an acute ischemic stroke, the location and size of the thrombus T may be identified using contrast media and medical imaging (e.g., magnetic resonance imaging (MRI), computed tomography (CT)). Certain dimensions of the double flow catheter 102 to be used in the procedure may be selected based on the location and size of the thrombus T determined from the initial imaging. For example, the length of the nose 124 may be selected to be long enough to cross the thrombus T such that the first orifice 117 may be positioned proximal to the thrombus T while the second orifice 126 is positioned distal to the thrombus T. As another example, the maximum radial dimension of the outer surface 130 of the body 128 may be selected to fit within the vessel at the location of the thrombus T. As still another example, given that the first radial cross-sectional area of the first lumen 115 must accommodate the thrombus T to be removed and the maximum radial dimension of the outer surface 130 of the body 128 is determined by the size of the vessel at the location of the thrombus T, the first radial cross-sectional area of the first lumen 115 and/or the second radial cross-sectional area of the second lumen 122 may be selected based on the size of the thrombus T. Several examples of dimensional configurations of the double flow catheter 102 are provided above in relation to thrombi found in various parts of the neurovasculature.

A micro-guidewire may be introduced into the arterial network of the patient (e.g., through the femoral artery) and navigated to the location of the thrombus T in the neurovasculature. The micro-guidewire may extend through the second orifice 126 defined by the nose 124 and into second lumen 122 of the double flow catheter 102. The nose 124 may be pushed along the micro-guidewire such that the nose 124 - and, thus, the body 128 of the double flow catheter 102 - follows the micro-guidewire to the location of the thrombus T in the neurovasculature.

At the location of the thrombus T, the nose 124 may cross the thrombus T by following the micro-guidewire to move between the thrombus T and a wall W of the vessel. The nose 124 may be advanced distal to the thrombus T until the second orifice 126 is distal to the thrombus T while the first orifice 117 is proximal to the thrombus T.

With the first orifice 117 and the second orifice 126 properly positioned relative to the thrombus T, treatment may be carried out to remove the thrombus T. For example, the vacuum source 104 may be actuated (e.g., by withdrawing a plunger of a syringe from an end-of-travel position) to apply suction to the thrombus T via the first orifice 117 and the first lumen 115 proximal to the thrombus T. To resist vessel collapse that may lead to severe brain injury and to facilitate moving the thrombus T back toward the first orifice 117, liquid (e.g., saline with or without lyse drug) at a controlled pressure may be injected distal to the thrombus via the second orifice 126 and the second lumen 122 while suction is being applied proximal to the thrombus T. For example, blood pressure of the patient may be measured, and a pouch may be inflated about an IV bag to a pressure substantially matching the blood pressure of the patient or to a maximum pressure of about 300 mmHg. In this context, the terms "substantially" and "about" shall be understood to reflect small fluctuations that may be attributable to normal anatomical variations in combination, in some instances, with inaccuracies that associated with manually controlling injection.

In certain implementations, with the nose 124 in place distal to the thrombus T, the fluid communication between the second lumen 122 and the vessel may be used to make a diagnosis of the state of the blood pressure distal to the thrombus T (e.g., using a pressure gauge or sensor in fluid communication with the second lumen 122 and outside of the body of the patient). Further, or instead, with the nose 124 in place distal to the thrombus T, the second lumen 122 may be used to introduce brain protection material and/or thrombus lysis material distal to the thrombus T.

In instances in which the application of suction through the first lumen 115 does not remove the thrombus T while injection is performed distal to the thrombus through the nose 124, the double flow catheter 102 may advantageously facilitate the use of additional removal techniques without requiring withdrawal of the double flow catheter 102. More specifically, in the treatment of acute ischemic stroke, the time required to remove the thrombus T is generally an important factor in achieving favorable outcomes (e.g., preservation of brain function). Because additional devices (e.g., a stent retriever) may be delivered to the treatment site through the large size of the first cross-sectional area of the first lumen 115, more favorable patient outcomes may be possible as compared to treatment techniques requiring time to withdraw a catheter before additional devices may be delivered to the treatment site.

While certain implementations have been described, other implementations are additionally or alternatively possible.

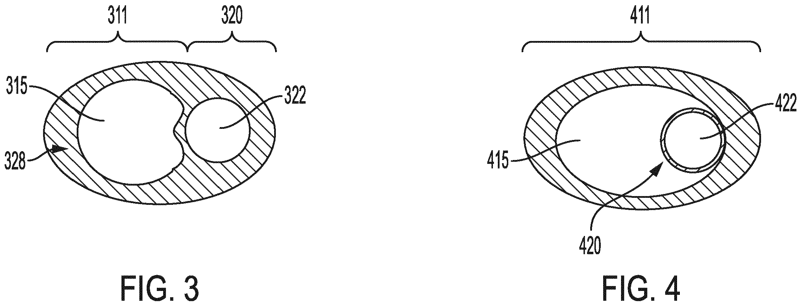

For example, while catheters have been described as having a certain type adjacency of a first lumen and a second lumen, other types of adjacency are additionally or alternatively possible. As an example, referring now to FIG. 3, a first tube 311 and a second tube 320 coupled to one another may form a body 328. For the sake of clear and efficient description, elements with 300-series numbers shall be understood to be analogous to or interchangeable with elements with 100-series element numbers (e.g., in FIGS. 1A-1D) described herein, unless otherwise explicitly made clear from the context and, therefore, are not described separately from counterpart 100-series element numbers, except to note certain differences and/or to emphasize certain features.

The first tube 311 may define a first lumen 315, and the second tube 320 may define a second lumen 322. The shape of the first lumen 315 may match the shape of at least a portion of the second tube 320 such that the first radial cross-sectional area of the first lumen 315 has a kidney-like shape. As compared to other shapes described herein, for otherwise identical geometry, the kidney-like shape of the first radial cross-sectional area of the first lumen 315 may facilitate achieving a larger radial cross-sectional area of the first lumen 315, as compared to a circular shape.

As another example, referring now to FIG. 4, a first tube 411 and a second tube 420 coupled to one another may form a body 428. For the sake of clear and efficient description, elements with 400-series numbers shall be understood to be analogous to or interchangeable with elements with 100-series element numbers (e.g., in FIGS. 1A-1D) described herein, unless otherwise explicitly made clear from the context and, therefore, are not described separately from counterpart 100-series element numbers, except to note certain differences and/or to emphasize certain features.

The first tube 411 may define a first lumen 415 and the second tube 420 may define a second lumen 422. The second tube 420 may be disposed in the first lumen 415 and coupled to the first tube 411 along a line of contact between the first tube 411 and the second tube 420.

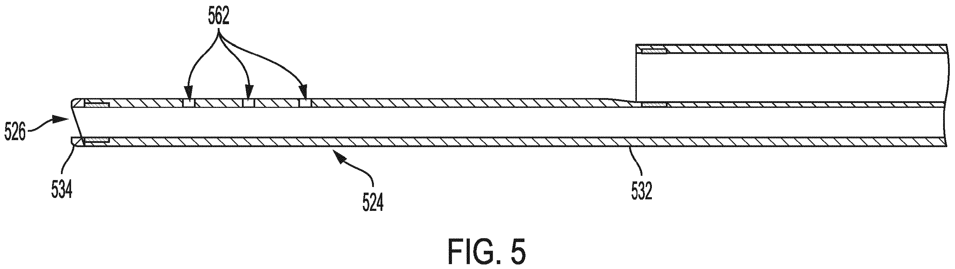

As another example, while catheters have been described as including noses with a single orifice, other openings in noses of catheters are additionally or alternatively possible. For example, referring now to FIG. 5, a nose 524 may have a proximal section 532 and a distal section 534, and the distal section 534 may define a second orifice 526. For the sake of clear and efficient description, elements with 500-series numbers shall be understood to be analogous to or interchangeable with elements with 100-series element numbers (e.g., in FIGS. 1A-1D) described herein, unless otherwise explicitly made clear from the context and, therefore, are not described separately from counterpart 100-series element numbers, except to note certain differences and/or to emphasize certain features.

The nose 524 may define a plurality of apertures 562 from the proximal section 532 to the distal section 534. The plurality of apertures 562 may be positioned along the nose 524 to direct injected liquid away from the nose 524 in a radial direction. For example, the plurality of apertures 562 may be positioned along the nose 524 to direct injected liquid toward a centerline of the artery, as may be useful for making efficient use of the injected liquid in reducing the likelihood of collapse of the vessel.

The method steps of the implementations described herein are intended to include any suitable method of causing such method steps to be performed, consistent with the patentability of the following claims, unless a different meaning is expressly provided or otherwise clear from the context. So, for example performing the step of X includes any suitable method for causing another party such as a remote user, a remote processing resource (e.g., a server or cloud computer) or a machine to perform the step of X. Similarly, performing steps X, Y and Z may include any method of directing or controlling any combination of such other individuals or resources to perform steps X, Y and Z to obtain the benefit of such steps. Thus, method steps of the implementations described herein are intended to include any suitable method of causing one or more other parties or entities to perform the steps, consistent with the patentability of the following claims, unless a different meaning is expressly provided or otherwise clear from the context. Such parties or entities need not be under the direction or control of any other party or entity, and need not be located within a particular jurisdiction.

It will be appreciated that the methods and systems described above are set forth by way of example and not of limitation. Numerous variations, additions, omissions, and other modifications will be apparent to one of ordinary skill in the art. Absent an explicit indication to the contrary, the disclosed steps may be modified, supplemented, omitted, and/or re-ordered without departing from the scope of this disclosure. In addition, the order or presentation of method steps in the description and drawings above is not intended to require this order of performing the recited steps unless a particular order is expressly required or otherwise clear from the context. Thus, while particular embodiments have been shown and described, it will be apparent to those skilled in the art that various changes and modifications in form and details may be made therein without departing from the spirit and scope of this disclosure and are intended to form a part of the invention as defined by the claims.

Additional Clauses - the following are a list of numbered clauses forming part of the description.
1. A double flow catheter for removal of an occlusion within a vessel, the double flow catheter comprising:
   a first tube having a proximal portion and a distal portion and defining first lumen therebetween, the distal portion defining a first orifice in fluid communication with the first lumen, the first lumen having a first radial cross-sectional area; and
   a second tube defining a second lumen parallel to the first lumen, the second tube including a nose defining a portion of the second lumen distal to the first lumen, the nose defining a second orifice in fluid communication with the second lumen, the second tube and the first tube coupled to one another and collectively defining a body proximal to the nose, and the second lumen having a second radial cross-sectional area less than the first radial cross-sectional area.
2. The double flow catheter of clause 1, wherein the body has an overall bend radius that decreases from the proximal portion to the distal portion of the first tube.
3. The double flow catheter of clause 2, wherein the overall bend radius decreases according to a gradient along at least a portion of a longitudinal dimension of the body.
4. The double flow catheter of any one of the preceding clauses, wherein the first lumen defines a first longitudinal axis, the second lumen defines a second longitudinal axis, and the first longitudinal axis and the second longitudinal axis are parallel to one another and radially spaced from one another at least from the proximal portion of the first tube to the distal portion of the first tube.
5. The double flow catheter of any one of the preceding clauses, wherein the second radial cross-sectional area of the second lumen is proximal to the nose of the second tube.
6. The double flow catheter of clause 5, wherein the second radial cross-sectional area of the second lumen is along the portion of the second lumen defined by the nose of the second tube.
7. The double flow catheter of clause 6, wherein the second radial cross-sectional area is substantially constant along a longitudinal dimension of the second lumen.
8. The double flow catheter of any one of the preceding clauses, wherein the first radial cross-sectional area of the first lumen is substantially constant along a longitudinal dimension of the first lumen.
9. The double flow catheter of any one of the preceding clauses, wherein, at least from the proximal portion to the distal portion of the first tube, a ratio of the first radial cross-sectional area to the second radial cross-sectional area is less than about 11:1.
10. The double flow catheter of any one of the preceding clauses, wherein the second radial cross-sectional area is greater than or equal to 0.1 mm² and less than or equal to 0.25 mm².
11. The double flow catheter of any one of the preceding clauses, wherein the shape of the first radial cross-sectional area of the first lumen and the shape of the second radial cross-sectional area of the second lumen each include a respective arcuate segment.
12. The double flow catheter of clause 11, wherein the shape of the first radial cross-sectional area of the first lumen is elliptic from the proximal portion to the distal portion of the first tube.
13. The double flow catheter of any one of clauses 11 or 12, wherein the shape of the second radial cross-sectional area of the second lumen is elliptic
14. The double flow catheter of any one of the preceding clauses, wherein the second lumen is fluidically isolated from the first lumen.
15. The double flow catheter of any one of the preceding clauses, wherein the body has an outer surface continuous from the proximal portion to the distal portion of the first tube.
16. The double flow catheter of clause 15, wherein the outer surface of the body has a maximum radial dimension greater than about 1 mm and less than about 6 mm.
17. The double flow catheter of clause 16, wherein, from the proximal portion to the distal portion of the first tube, a circumference of the outer surface of the body has an oblong shape.
18. The double flow catheter of clause 17, wherein, from the proximal portion to the distal portion of the first tube, the body is more flexible in a first plane defined by a minor dimension of the oblong shape and a longitudinal axis defined by the first lumen than in a second plane defined by a major dimension of the oblong shape and the longitudinal axis defined by the first lumen.
19. The double flow catheter of any one of clauses 17 or 18, wherein, from the proximal portion to the distal portion of the first tube, the circumference of the outer surface is stadium-shaped.
20. The double flow catheter of any one of clauses 17-19, wherein, from the proximal portion to the distal portion of the first tube, a circumference of the outer surface is oval.
21. The double flow catheter of clauses 15-20, wherein the outer surface of the body includes a hydrophilic coating.
22. The double flow catheter of any one of the preceding clauses, further comprising a first lining in the first lumen and a second lining in the second lumen, wherein the first lining and the second lining are each hydrophobic.
23. The double flow catheter of clause 22, wherein at least one of the first lining or the second lining includes polytetrafluoroethylene (PTFE), a polymer having a lower surface energy than PTFE, or a combination thereof.
24. The double flow catheter of any one of the preceding clauses, wherein the first tube has a first radial compressive strength of at least about 1050 mmHg along the first lumen, and the second tube has a burst pressure of at least about 1050 mmHg along the second lumen at least proximal to the nose of the second tube.
25. The double flow catheter of any one of the preceding clauses, wherein at least one of the first tube or the second tube has a burst pressure of greater than 37500 mmHg.
26. The double flow catheter of any one of the preceding clauses, wherein the first tube and the second tube are fused to one another from the proximal portion to the distal portion of the first tube.
27. The double flow catheter of any one of the preceding clauses, wherein the first tube has a first polymeric composition profile varying in polymeric composition in an axial direction along the first tube, and the second tube has a second polymeric composition profile varying in polymeric composition in an axial direction along the second tube.
28. The double flow catheter of clause 27, wherein the first polymeric composition profile and the second polymeric composition profile have the same composition in at least one axial position along a longitudinal dimension of the body from the proximal portion to the distal portion of the first tube.
29. The double flow catheter of clauses 27 or 28, wherein the nose of the second tube includes a proximal section and a distal section, the proximal section of the nose is adjacent to the distal portion of the first tube, and the distal section of the nose is distally away from the distal portion of the first tube and defines the second orifice, and the second polymeric composition profile is softest along at least a portion of the nose between the proximal section and the distal section of the nose.
30. The double flow catheter of clause 29, wherein composition of the second polymeric composition profile is constant in an axial direction from the proximal section to the distal section of the nose.
31. The double flow catheter of any one of clauses 29 or 30, wherein the nose has a longitudinal dimension of greater than about 15 mm and less than about 75 mm from the proximal section to the distal section.
32. The double flow catheter of any one of clauses 29-31, wherein, from the proximal section to the distal section, a maximum outer dimension of the nose is greater than about 0.4 mm and less than about 1.0 mm.
33. The double flow catheter of any one of clauses 29-32, wherein, from the proximal section to the distal section, an outer circumference of the nose is circular.
34. The double flow catheter of any one of clauses 29-33, wherein the portion of the second lumen defined by the nose accommodates passage of a micro guidewire from the proximal section of the nose through the second orifice defined by the distal section of the nose.
35. The double flow catheter of clause 34, wherein the portion of the second lumen defined by the nose has a maximum radial inner dimension of greater than about 0.2 mm and less than about 0.6 mm.
36. The double flow catheter of any one of clauses 29-35, wherein the nose defines a plurality of apertures from the proximal section to the distal section.
37. The double flow catheter of the preceding clauses, wherein the body includes one or more reinforcement materials from the proximal portion to the distal portion of the first tube.
38. The double flow catheter of clause 37, wherein density of the one or more reinforcement materials per unit of axial length decreases in an axial direction from the proximal portion to the distal portion of the first tube.
39. The double flow catheter of clause 38, wherein the one or more reinforcement materials includes one or more wires.
40. The double flow catheter of clause 39, wherein at least one of the one or more wires are wrapped in a pattern with decreasing pitch, have decreasing cross-sectional area, or a combination thereof along the body in the axial direction from the proximal portion to the distal portion of the first tube.
41. The double flow catheter of clauses 39 or 40, wherein the one or more wires form a coil, an overlapping pattern, an interwoven pattern, or a combination thereof about at least one of the first tube or the second tube.
42. The double flow catheter of clauses 37-41, wherein the one or more reinforcement materials includes stainless steel, nitinol, tungsten, polymeric fibers, or a combination thereof.
43. The double flow catheter of the preceding clauses, further comprising a plurality of radiopaque markers, wherein at least one of the plurality of radiopaque markers is disposed along the distal portion of the first tube, and at least one of the plurality of radiopaque markers is disposed along the nose of the second tube.
44. The double flow catheter of clause 43, wherein the plurality of radiopaque markers include rings, dots, or a combination thereof of radiopaque material.
45. The double flow catheter of any one of clauses 43 or 44, wherein the radiopaque material includes one or more radiopaque polymers.

## Claims

1. A double flow catheter for removal of an occlusion within a vessel, the double flow catheter comprising:
a first tube having a proximal portion and a distal portion and defining first lumen therebetween, the distal portion defining a first orifice in fluid communication with the first lumen, the first lumen having a first radial cross-sectional area; and
a second tube defining a second lumen parallel to the first lumen, the second tube including a nose defining a portion of the second lumen distal to the first lumen, the nose defining a second orifice in fluid communication with the second lumen, the second tube and the first tube coupled to one another and collectively defining a body proximal to the nose, and the second lumen having a second radial cross-sectional area less than the first radial cross-sectional area.

2. The double flow catheter of clause 1, wherein the body has an overall bend radius that decreases from the proximal portion to the distal portion of the first tube.

3. The double flow catheter of clause 2, wherein the overall bend radius decreases according to a gradient along at least a portion of a longitudinal dimension of the body.

4. The double flow catheter of any one of the preceding clauses, wherein the first lumen defines a first longitudinal axis, the second lumen defines a second longitudinal axis, and the first longitudinal axis and the second longitudinal axis are parallel to one another and radially spaced from one another at least from the proximal portion of the first tube to the distal portion of the first tube.

5. The double flow catheter of any one of the preceding clauses, wherein the second radial cross-sectional area of the second lumen is proximal to the nose of the second tube.

6. The double flow catheter of clause 5, wherein the second radial cross-sectional area of the second lumen is along the portion of the second lumen defined by the nose of the second tube.

7. The double flow catheter of any one of the preceding clauses, wherein, at least from the proximal portion to the distal portion of the first tube, a ratio of the first radial cross-sectional area to the second radial cross-sectional area is less than about 11:1, and the second radial cross-sectional area is greater than or equal to 0.1 mm² and less than or equal to 0.25 mm².

8. The double flow catheter of any one of the preceding clauses, wherein the second lumen is fluidically isolated from the first lumen.

9. The double flow catheter of any one of the preceding clauses, wherein the body has an outer surface continuous from the proximal portion to the distal portion of the first tube.

10. The double flow catheter of clause 9, wherein the outer surface of the body has a maximum radial dimension greater than about 1 mm and less than about 6 mm; and, from the proximal portion to the distal portion of the first tube, a circumference of the outer surface of the body has an oblong shape, wherein, from the proximal portion to the distal portion of the first tube, the body is more flexible in a first plane defined by a minor dimension of the oblong shape and a longitudinal axis defined by the first lumen than in a second plane defined by a major dimension of the oblong shape and the longitudinal axis defined by the first lumen.

11. The double flow catheter of any one of the preceding clauses, further comprising a first lining in the first lumen and a second lining in the second lumen, wherein the first lining and the second lining are each hydrophobic.

12. The double flow catheter of any one of the preceding clauses, wherein the first tube has a first radial compressive strength of at least about 1050 mmHg along the first lumen, and the second tube has a burst pressure of at least about 1050 mmHg along the second lumen at least proximal to the nose of the second tube.

13. The double flow catheter of any one of the preceding clauses, wherein the first tube and the second tube are fused to one another from the proximal portion to the distal portion of the first tube.

14. The double flow catheter of any one of the preceding clauses, wherein:
the first tube has a first polymeric composition profile varying in polymeric composition in an axial direction along the first tube, and the second tube has a second polymeric composition profile varying in polymeric composition in an axial direction along the second tube;
the first polymeric composition profile and the second polymeric composition profile have the same composition in at least one axial position along a longitudinal dimension of the body from the proximal portion to the distal portion of the first tube;
the nose of the second tube includes a proximal section and a distal section, the proximal section of the nose is adjacent to the distal portion of the first tube, and the distal section of the nose is distally away from the distal portion of the first tube and defines the second orifice; and
the second polymeric composition profile is softest along at least a portion of the nose between the proximal section and the distal section of the nose.

15. The double flow catheter of the preceding clauses, wherein:
the body includes one or more reinforcement materials from the proximal portion to the distal portion of the first tube;
density of the one or more reinforcement materials per unit of axial length decreases in an axial direction from the proximal portion to the distal portion of the first tube; and
the one or more reinforcement materials includes one or more wires.
